Europäisches Patentamt

European Patent Office

Office européen des brevets

(19)

(11) Publication number: **0 275 204**
**A2**

# (12) EUROPEAN PATENT APPLICATION

(21) Application number: **88300303.0**

(22) Date of filing: **14.01.88**

(51) Int. Cl.⁴: **C 12 N 15/00**
**C 12 P 21/02**

(30) Priority: **16.01.87 US 4379    03.11.87 US 116430**

(43) Date of publication of application:
**20.07.88 Bulletin 88/29**

(84) Designated Contracting States:
**AT BE CH DE ES FR GB GR IT LI LU NL SE**

(71) Applicant: **Amgen Inc.**
**1900 Oak Terrace Lane**
**Thousand Oaks, California 91320 (US)**

(72) Inventor: **Banks, Allen Rush**
**2886 Middlefork Road**
**Boulder Colorado 80302 (US)**

**Fox, Gary Michael**
**11 Dorrit Court**
**Newbury Park California 91320 (US)**

(74) Representative: **Brown, John David et al**
**FORRESTER & BOEHMERT Widenmayerstrasse 4/I**
**D-8000 München 22 (DE)**

(54) Production of fibroblast growth factor.

(57) Disclosed is a process for producing E. coli derived recombinant fibroblast growth factor possessing part or all of the primary structural conformation and one or more of the biological properties of a mammalian (e.g., human) basic fibroblast growth factor which process is characterized as the host expression of an exogenous DNA sequence. Sequences coding for part or all of the sequence of amino acid residues of bFGF may be incorporated in autonomously replicating plasmid or viral vectors having a regulated promoter sequence and a temperature inducible copy number control gene employed to transform suitable procaryotic host cells such as E. coli in culture.

EP 0 275 204 A2

Description

## PRODUCTION OF FIBROBLAST GROWTH FACTOR

The present invention relates to a method for producing recombinant basic fibroblast growth factor in an E. coli host strain. In particular, the present invention refers to a method for producing recombinant human basic fibroblast growth factor ("human r-bFGF") in an E. coli host strain and to polynucleotides encoding such factors.

### BACKGROUND

Fibroblast Growth Factor (FGF) was first described by Gospodarowicz, Nature, 249, 123 (1974) as an activity derived from bovine brain or pituitary tissue which was mitogenic for fibroblasts and endothelial cells. It was later noted that the primary mitogen from brain was different than that isolated from pituitary. These two factors were named acidic and basic FGF because they had similar if not identical biological activities but differed in their isoelectric points. Subsequently other endothelial cell mitogens were isolated from a number of tissues and tumors which are very similar or identical to basic FGF. Such factors include, for example, hepatoma-derived growth factorm (Klagsbrun, et al, PNAS, 83, 2448-2452 (1986) and Lobb et al, J.Biol.Chem., 23, 6295-6299 (1984)), chondrosarcoma-derived growth factor (Shing et al, Science, 223, 1296-1299 (1984)), beta retina-derived growth factor (Baird et al, Biochemistry, 24, 7855-7860 (1985)), cartilage-derived growth factor (Sullivan and Klagsbrun, J.Biol. Chem., 260, 2399-2403 (1985)), astroglial growth factor 2 (Pettman et al, FEBS Lett., 189, 102-108), eye-derived growth factor (Courty et al, Biochimie, 67, 265-2698 (1985)), cationic hypothalamus-derived growth factor Klagsbrun and Shing, PNAS, 82, 805-809 (1985)), class 2 and beta heparin-binding growth factors (Lobb and Fett, Biochemistry, 23, 6265-6299 (1984); Lobb et al, Biochem., 24, 4969-4973 (1985); Lobb et al, BBRC, 131, 586-592 (1985); Lobb et al, J.Biol.Chem., 261, 1924-1928 (1986)), and a component of macrophage-derived growth factor (Baird et al, BBRC, 126, 358-364 (1985)). All of the above factors share basic FGF's property of binding tightly to heparin and all are basic proteins. A similar group of heparin-binding factors, typified by acidic FGF, have also been found. These molecules elute from heparin at a lower sodium chloride concentration and have acidic isoelectric points. The heparin binding property of these factors has facilitated their purification, allowing isolation of sufficient protein for amino acid sequence analysis in several cases. Although the use of heparin has facilitated purification of FGF, the use of heparin in large scale purification procedures is undesirable because of the expense, possible contamination of product with heparin, and loss of yield due to irreversible binding to heparin. Acidic and basic FGF are probably derived from the same ancestral gene and are 55% homologous in amino acid sequence and have the same intron/exon structure. Southern blotting experiments suggest that there is only one gene each for acidic and basic FGF; differences between the molecules isolated from different tissues are probably due to post-translational processing. The range of biological activities of the two classes appears to be identical, although basic FGF is about ten times more potent than acidic FGF in most bioassay systems.

Basic FGF is a single chain, non-glycosylated protein having a molecular weight of approximately 16,500. Basic FGF contains four cysteine residues, but the number of disulfide bonds, if any, is unknown. A primary translation product having 155 amino acids has been proposed for basic FGF, but the major form found in pituitary tissue has 146 amino acids. Several molecular weight forms, differing in length at the N-terminus, have been isolated from different tissues, all of which appear to retain biological activity. FGF is an extremely basic protein, with an isoelectric point of 9.6 Basic FGF binds avidly to heparin, eluting from heparin sepharose columns at around 1.6 M NaCl. The biological activity of FGF is destroyed by heat (70°C) or by detergents. The protein appears to be stable at 37°C for fairly long periods. In the genome, coding sequences for this translation product are interrupted by two introns; the first splits codon 60 and the second separates codons 94 and 95. The size of the entire genomic coding region is not known, but it is at least 34 kb in length. The gene for basic FGF is located on chromosome 4.

The first sequence data for basic FGF was published by Bohlen et al PNAS, 81, 5364-5368, (1984) who determined the N-terminal 15 amino acids of material purified from bovine pituitary tissue. Subsequently, Esch et. al., PNAS, 82, 6507-6511, (1985) reported the complete sequence of basic FGF from bovine pituitary and at the same time compared it with amino terminal sequence from acidic FGF. PCT patent application WO 86/07595 discloses the production of bovine bFGF in E. coli. However, the reported yields of product are extremely low. Cloning of the gene for bovine basic FGF was first reported by Abraham et al Science, 233, 545-548, and a later paper by the same authors described the nucleotide sequence and genomic organization of human basic FGF (EMBO Journal, 5, 2523-2528 (1986)). Bovine and human basic FGF are known to differ only by two amino acids.

Although highly purified preparations of basic FGF have only recently been available for testing, many in vitro studies have been published using material of various states of purity. In these studies, FGF has been shown to be a potent mitogen for a wide variety of cells of mesodermal origin and may be chemotactic for endothelial cells and fibroblasts. In addition, naturally occurring and tissue derived recombinant basic FGF appears to induce neovascularization in both the rabbit cornea and chick chorioallantoic membrane assays, thus FGF may be useful in accelerating the healing of wounds. Fourtanier et al, J.Inv.Derm., 87, 76-80 (1986) disclosed that a preparation derived from bovine retina was able to stimulate neovascularization and reepithelialization and to promote the healing of wounds in a guinea pig blister model. Davidson et al, J.Cell.Biol., 100, 1219-1227 (1985) have shown accelerated wound repair accompanied by increased granulation tissue and collagen accumulation to be induced by a bovine cartilage derived factor in a rat wound model system. Buntrock and coworkers Exp.Path., 21, 46-53, and Exp.Path., 21, 62-67 (1982) have also reported increases in granulation tissue and neovascularization along with accelerated healing of wounds in rats using an extract of bovine brain tissue.

## SUMMARY OF THE INVENTION

The present invention relates to a process for producing a polypeptide having part or all of the primary structural conformation and one or more of the biological properties of naturally occurring human basic fibroblast growth factor, said process comprising:

1) growing under suitable nutrient conditions, E. coli host cells transformed with a DNA plasmid vector wherein the DNA plasmid vector comprises a DNA sequence coding for E. coli host expression of a polypeptide having part of all of the primary structural conformation and one or more of the biological properties of human basic fibroblast growth factor, a regulated promoter sequence, and a temperature inducible copy number control gene;

2) isolating desired polypeptide products of the expression of DNA sequences in said vector;

3) purifying the desired polypeptide product.

The present invention further relates to a method for purifying E. coli derived recombinant basic fibroblast growth factor using a non-heparin containing chromatographic system.

According to the present invention, DNA sequences coding for all or part of human basic fibroblast growth factor are provided. Such sequences preferably may include, the incorporation of codons "preferred' for expression by selected E. coli host strains ("E. coli cleavage by restriction endonuclease enzymes; and the provision of additional initial, terminal or intermediate DNA sequences which facilitate construction of readily expressed vectors. The novel DNA sequences of the invention include sequences useful in securing expression in E. coli host cells of polypeptide products having at least a part of the primary structural conformation and one or more of the biological properties of naturally occurring basic fibroblast growth factor. DNA sequences of the invention are specifically seen to comprise: (a) the DNA sequence set forth in Table II or the complimentary strands; (b) a DNA sequence which hybridizes (under hybridization conditions such as illustrated herein or more stringent conditions) to the DNA sequences in Table II or to fragments thereof; and (c) a DNA sequence which, but for the degeneracy of the genetic code, would hybridize to the DNA sequence in Table VII. Specifically comprehended by part (c) are manufactured DNA sequences encoding basic fibroblast growth factor which DNA sequences may incorporate codons facilitating translation of messenger RNA in microbial hosts. Such manufactured sequences may readily be constructed according to the methods of Alton, et. al., PCT published application WO 83/04053.

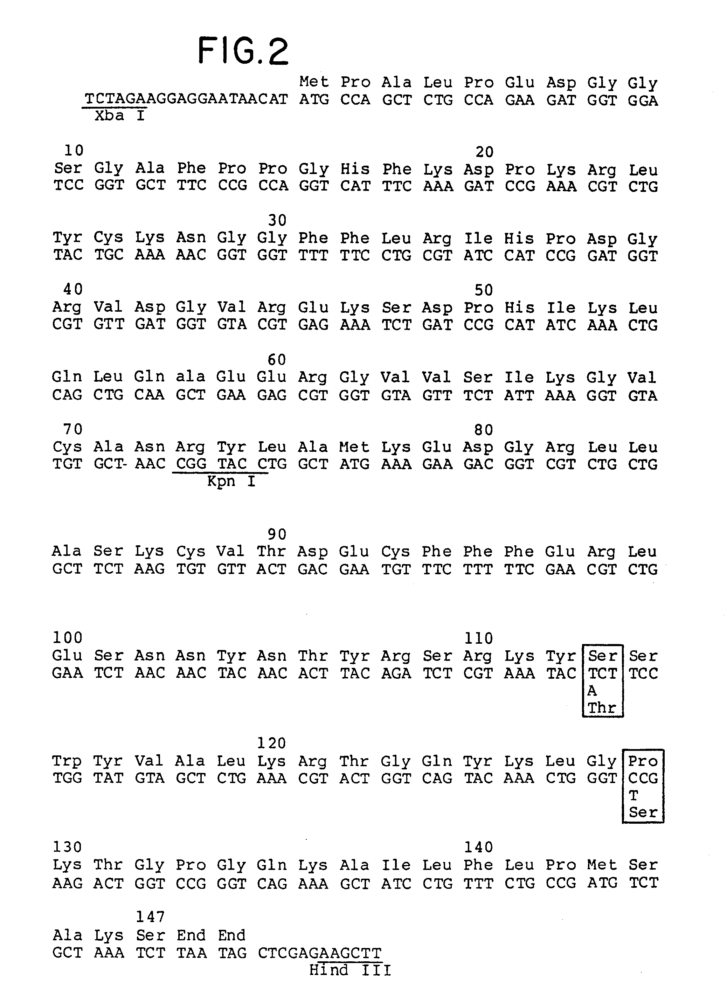

The present invention provides purified and isolated polypeptide products having part or all of the primary structural conformation (i.e., continuous sequence of amino acid residues) and one or more of the biological properties (e.g., immunological properties and in vitro biological activity) and physical properties (e.g., molecular weight) of naturallyoccurring basic fibroblast growth factor including allelic variants thereof. These polypeptides are also characterized by being the product of E. coli host expression of exogenous DNA sequences obtained by gene synthesis. The polypeptide products of the present invention expressed from E. coli host cells are free of association with any mammalian proteins. In addition, polypeptides of the present invention may also include an initial methionine amino acid residue (at position 1 as shown in Fig. 2).

The present invention also encompasses the various replicable cloning vehicles, expression vehicles and transformed E. coli cultures, all harboring the altered genetic information necessary to affect the production of E. coli derived recombinant basic fibroblast growth factor.

Numerous aspects and advantages of the invention will be apparent to those skilled in the art upon consideration of the following detailed description which provides illustrations of the practice of the invention in its presently preferred embodiments.

Brief Description of the Drawings

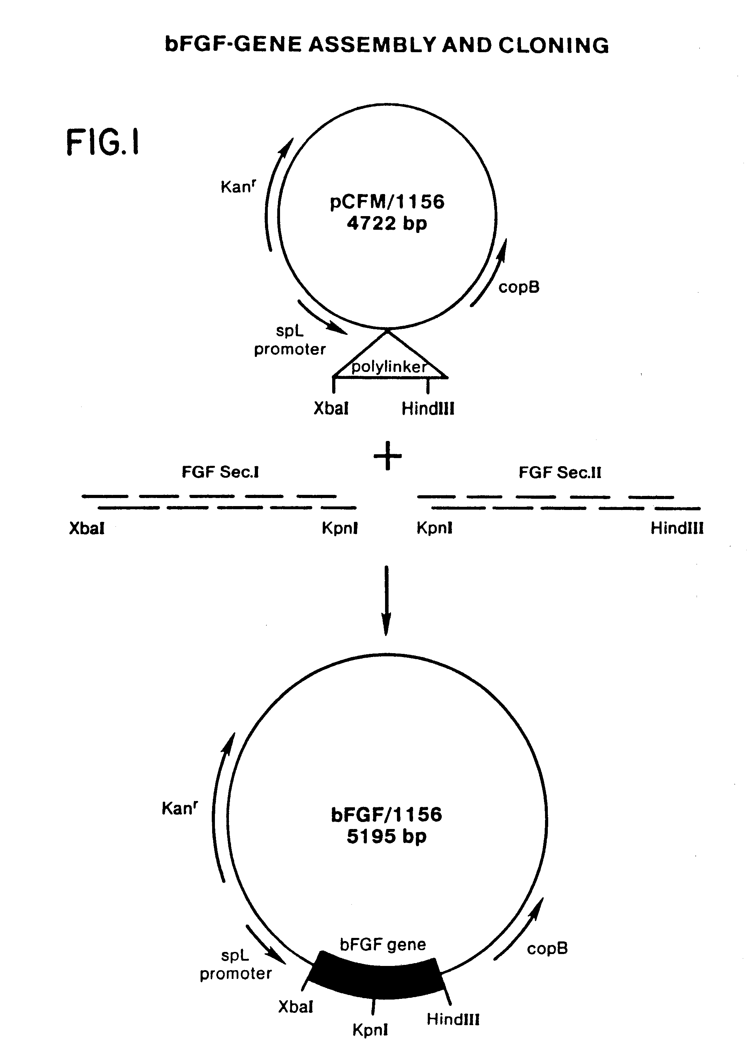

Fig. 1 is a diagrammatic representation of the bFGF gene assembly and cloning.

Fig. 2 is the nucleotide and amino acid sequences of recombinant bFGFs. The solid boxes outline the nucleotide and resultant amino acid changes which were produced by site-directed mutagenesis in order to convert the bovine gene to one coding for human bFGF.

Fig. 3 is a graph of the mitogenic activity of bFGFs on NIH3T3 cells. The mitogenic effect of human bFGF (●) and bovine bFGF (o) on NIH3T3 cells is shown. The dose of bFGF is plotted against the percentage of maximal stimulation of DNA synthesis as measured by 3H thymidine uptake at that dose.

## DETAILED DESCRIPTION

As used herein, the term "tissue-derived basic fibroblast growth factor" refers to basic fibroblast growth factor derived from tumors, eucaryotic cells maintained in culture, normal tissues and the like.

As employed herein, the term "manufactured" as applied to a DNA sequence or gene shall designate a product either totally chemically synthesized by assembly of nucleotide bases or derived from the biological replication of a product thus chemically synthesized. As such, the term is exclusive of products "synthesized" by cDNA methods of genomic cloning methodologies which involve starting materials which are initially of biological origin.

As used herein, the term "allelic versions" refers to modifications of one or more amino acids in the sequence of the bFGF analogs of the present invention without altering the biological activity of the analog. Such allelic versions are readily ascertained by one of ordinary skill in the art.

The E. coli derived recombinant basic FGF was produced in accordance with the following general procedure:

The amino acid sequence of bovine basic FGF published by Esch et al PNAS, 82, 6507-6511 (1985) was used as a basis for the synthesis of manufactured bFGF gene for expression in E. coli. The nucleotide sequence of this manufactured gene includes codons most often used by E. coli and the inclusion of convenient restriction sites to be used for cloning purposes. Illustrative of the manufactured genes includes the gene represented in Table I and Table II. Table I represents a manufactured gene for bovine bFGF and Table II represents a manufactured gene for human bFGF. Oligonucleotides corresponding to both strands of the gene were synthesized in overlapping sections and assembled into two larger sections by hybridization and subsequent ligation. The two larger sections were then cloned into an appropriate phage vector (i.e., M 13mp18) for nucleotide sequence analysis. Such phage vectors are readily ascertained by one of ordinary skill in the art. Upon verification of the correct sequence, both sections were excised by restriction endonuclease digestion, gel isolated, and ligated into an appropriate expression vector. Expression of the bFGF gene encoded on the plasmid is regulated by a regulated promoter sequence and the temperature inducible copy number control genes located on the expression vector. The term "regulated promoters" as used herein, refers to $P_L$ promoters and foreshortened $P_L$ promoter. Expression vectors containing such regulated promoters and temperature inducible copy number control genes are described in European Patent Application No. 136,490. Growth of this plasmid in an E. coli host strain yielded the polypeptide product of the present invention. When the bFGF containing cells are lysed and subjected to low speed centrifugation, about 70% of the FGF is found in soluble form in the supernatant fraction. Purification using non-heparin containing chromatographic systems, i.e., affinity chromatography results in a polypeptide product which is estimated to be at least 95% pure by polyacrylamide gel electrophoresis and contains virtually no endotoxin or DNA contamination. The use of non-heparin containing chromatographic system in the purification of bFGF thus enables one to increase yields and eliminate possible product contamination due to heparin.

## TABLE I

Bovine basic Fibroblast Growth Factor/manufactured gene

```
          10                  30                  50
CTAGAAGGAGGAATAACATATGCCAGCTCTGCCAGAAGATGGTGGATCCGGTGCTTTCCC
GATCTTCCTCCTTATTGTATACGGTCGAGACGGTCTTCTACCACCTAGGCCACGAAAGGG

          70                  90                 110
GCCAGGTCATTTCAAAGATCCGAAACGTCTGTACTGCAAAAACGGTGGTTTTTTCCTGCG
CGGTCCAGTAAAGTTTCTAGGCTTTGCAGACATGACGTTTTTGCCACCAAAAAAGGACGC

         130                 150                 170
TATCCATCCGGATGGTCGTGTTGATGGTGTACGTGAGAAATCTGATCCGCATATCAAACT
ATAGGTAGGCCTACCAGCACAACTACCACATGCACTCTTTAGACTAGGCGTATAGTTTGA

         190                 210                 230
GCAGCTGCAAGCTGAAGAGCGTGGTGTAGTTTCTATTAAAGGTGTATGTGCTAACCGGTA
CGTCGACGTTCGACTTCTCGCACCACATCAAAGATAATTTCCACATACACGATTGGCCAT

         250                 270                 290
CCTGGCTATGAAAGAAGACGGTCGTCTGCTGGCTTCTAAGTGTGTTACTGACGAATGTTT
GGACCGATACTTTCTTCTGCCAGCAGACGACCGAAGATTCACACAATGACTGCTTACAAA

         310                 330                 350
CTTTTTCGAACGTCTGGAATCTAACAACTACAACACTTACAGATCTCGTAAATACTCTTC
GAAAAAGCTTGCAGACCTTAGATTGTTGATGTTGTGAATGTCTAGAGCATTTATGAGAAG

         370                 390                 410
CTGGTATGTAGCTCTGAAACGTACTGGTCAGTACAAACTGGGTCCGAAGACTGGTCCGGG
GACCATACATCGAGACTTTGCATGACCAGTCATGTTTGACCCAGGCTTCTGACCAGGCCC

         430                 450                 470
TCAGAAAGCTATCCTGTTTCTGCCGATGTCTGCTAAATCTTAATAGCTCGAGAAGCTT
AGTCTTTCGATAGGACAAAGACGGCTACAGACGATTTAGAATTATCGAGCTCTTCGAA
```

## TABLE II

Human basic Fibroblast Growth Factor/manufactured gene

```
        10              30              50
CTAGAACGAGGAATAACATATGCCAGCTCTGCCAGAAGATGGTCGATCCGCTGCTTTCCC
GATCTTCCTCCTTATTGTATACGGTCGAGACGGTCTTCTACCACCTAGGCCACGAAAGCG

        70              90              110
GCCAGGTCATTTCAAAGATCCGAAACGTCTGTACTGCAAAAACGGTGGTTTTTTCCTGCG
CGGTCCAGTAAAGTTTCTAGGCTTTGCAGACATGACGTTTTTGCCACCAAAAAAGGACGC

        130             150             170
TATCCATCCGGATGGTCGTGTTGATGGTGTACGTGAGAAATCTGATCCGCATATCAAACT
ATAGGTAGGCCTACCAGCACAACTACCACATGCACTCTTTAGACTAGGCGTATAGTTTGA

        190             210             230
GCAGCTGCAAGCTGAAGAGCCTGGTGTAGTTTCTATTAAAGGTGTATGTGCTAACCGGTA
CGTCGACGTTCGACTTCTCGCACCACATCAAAGATAATTTCCACATACACGATTGGCCAT

        250             270             290
CCTGGCTATGAAAGAAGACGGTCGTCTGCTGGCTTCTAAGTGTGTTACTGACGAATGTTT
GGACCGATACTTTCTTCTGCCAGCAGACGACCGAAGATTCACACAATGACTGCTTACAAA

        310             330             350
CTTTTTCGAACGTCTGGAATCTAACAACTACAACACTTACAGATCTCGTAAATACACTTC
GAAAAAGCTTGCAGACCTTAGATTGTTGATGTTGTGAATGTCTAGAGCATTTATGTGAAG

        370             390             410
CTGGTATGTAGCTCTGAAACGTACTGGTCAGTACAAACTGGGTTCGAAGACTGGTCCGGG
GACCATACATCGAGACTTTGCATGACCAGTCATGTTTGACCCAAGCTTCTGACCAGGCCC

        430             450             470
TCAGAAAGCTATCCTGTTTCTGCCGATGTCTGCTAAATCTTAATAGCTCGAGAAGCTT
AGTCTTTCGATAGGACAAAGACGGCTACAGACGATTTAGAATTATCGAGCTCTTCGAA
```

The r-bFGF polypeptide expressed from E. coli is purified using a chromatographic system that does not rely upon heparin for binding of the FGF. Preferably, the E. coli derived r-bFGF is purified using a non-heparin containing affinity chromatography column.

As previously mentioned, bovine and human bFGF differ by only two amino acids. Site-directed mutagenesis has been employed to convert the bovine gene into one coding for human bFGF. Since the human and bovine genes are so closely related, the purification scheme developed for bovine bFGF may be directly applicable to the human protein.

The mitogenic activity of the E. coli derived r-bFGF of the present invention was measured using a mitogenic assay based on the increase in radiolabeled thymidine uptake by mouse 3T3 cells which accompanies increased DNA synthesis during cell division. The angiogenic activity of the E. coli derived recombinant FGF was measured using a chick chorioallantoic membrane assay. Results from these experiments have shown that 1 µg of E. coli derived bovine r-bFGF of the present invention formulated in 0.45% methyl cellulose and dried shows no detectable angiogenic activity in the chick chorioallantoic membrane assay. Naturally-occurring bFGF formulated in the same manner and at the same dose exhibits angiogenic activity in the chick chorioallantoic membrane assay. However, the E. coli derived bovine r-bFGF formulated in the same manner at the same dose retains potent mitogenic activity on capillary endothelial cells as does naturally-occurring basic fibroblast growth factor. This surprising result may be due to many factors. The E. coli derived r-FGF polypeptide may be folded in a manner thereby enabling the polypeptide to retain mitogenic activity but not angiogenic activity. In addition, tissue-derived FGF preparations may contain contaminants which were responsible for tissue-derived FGF's reported angigenic properties. A third possibility is that some fragment of the primary FGF translation product is responsible for its angiogenic activity. It would be unlikely that any contaminant responsible for angiogenesis would be found in exactly the same range of tissues and would copurify in all cases unless it was a fragment of the original molecule.

The following examples serve to further illustrate the embodiments of the present invention:

## EXAMPLE 1

Preparation of a Manufactured Gene Encoding Bovine Basic FGF

This example relates to the preparation of a manufactured gene encoding bovine bFGF wherein E. coli expression preference codons are included. The protocol employed to prepare the manufactured gene encoding a recombinant FGF product is generally described in the disclosure of Alton, et al, PCT Publication No. W083/04053 which is incorporated by reference herein. The genes were designed for initial assembly of component oligonucleotides into multiple duplexes which, in turn, were assembled into 2 discrete sections. These sections were designed for ready amplification and, upon removal from the amplification system, could be assembled sequentially or through a multiple fragment ligation in a suitable expression vector.

Assembly of Section I of Fibroblast Growth Factor: 200pm of each of the 16 oligomers required for assembly of section I represented in Table III were measured into eppendorf tubes and dried on a speed vacuum pump. 320 µl of a kinase mix was prepared which contained 32 µl of 10 × ligation buffer (50M hepes, pH 7.6), 0.7 µl of 10 mM ATP, 1 µl of $3 \times 10^7$ counts/minute/ µl of radiolabelled ATP, and 266 µl of water. The reagents were combined in a tube of Boehringer Mannheim kinase which contained 20 µl of the kinase enzyme at a concentration of 10 units/µl. This kinase mix was aliquoted into each of tubes 2-15 containing oligonucleotides 2-15, respectively. Tubes containing oligonucleotides 1 and 16 received ligation buffer only. Tubes containing 2-15 were incubated at 37° for 45 minutes. At the end of that time period, 1/4 µl aliquots from each tube were spotted onto DE81 strips eluted with 0.35M ammonium formate and analyzed on a liquid scintillation counter. Liquid scintillation analysis showed that more than 1/2 of the counts were at the origin so 2 µl of 10mM ATP were added to each of the tubes containing 2-15 oligonucleotides and then the tubes were incubated an additional 45 minutes at 37°. At the end of this time period, all tubes were boiled for 10 minutes then centrifuged and combined into duplexes. This was done by adding tube 9 to tube 1 (duplex #1), tube 10 to tube 2 (duplex #2), tube 11 to tube 3 (duplex #3), tube 12 to tube 4 (duplex #4), tube 13 to tube 5 (duplex #5), tube 14 to tube 6 (duplex #6), tube 15 to tube 7 (duplex #7), and tube 16 to tube 8 (duplex # 8). These eight mixtures of oligonucleotides

## TABLE III

FGF OLIGOMERS, SECTION I

| | |
|---|---|
| CTAGAAGGAGGAATAACATATGCCAGCTCT | 1 |
| GCCAGAAGATGGTGGATCCGGTGCTTTCCC | 2 |
| GCCAGGTCATTTCAAAGATCCGAAACGTCTG | 3 |
| TACTGCAAAAACGGTGGTTTTTTCCTGCGTA | 4 |
| TCCATCCGGATGGTCGTGTTGATGGTGTAC | 5 |
| GTGAGAAATCTGATCCGCATATCAAACTGCA | 6 |
| GCTGCAAGCTGAAGAGCGTGGTGTAGTTT | 7 |
| CTATTAAAGGTGTATGTGCTAACCGGTACCTG | 8 |
| CTGGCAGAGCTGGCATATGTTATTCCTCCTT | 9 |
| TGGCGGGAAAGCACCGGATCCACCATCTT | 10 |
| AGTACAGACGTTTCGGATCTTTGAAATGACC | 11 |
| ATGGATACGCAGGAAAAAACCACCGTTTTTGC | 12 |
| TCACGTACACCATCAACACGACCATCCGG | 13 |
| CAGCTGCAGTTTGATATGCGGATCAGATTTC | 14 |
| ATAGAAACTACACCACGCTCTTCAGCTTG | 15 |
| AATTCAGGTACCGGTTAGCACATACACCTTTA | 16 |

were then boiled and slow cooled to room temperature to allow formation of the duplexes. The duplexes were then combined so that duplexes #1 and #2 were combined (tetramer 1 + 2), duplexes #3 and #4 (tetramer 3 + 4) were combined, duplexes #5 and #6 (tetramer 5 + 6) were combined, and finally duplexes #7 and #8 (tetramer 7 + 8) were combined. To each of these tubes, now containing tetramers, 2 μl of 10 mM ATP and 2 μl of T4 DNA ligase from Boehringer Mannheim were added. These ligation mixtures were then incubated for 10 minutes at 37° and then at room temperature for 1 hour. At this point, the tetrameric mixtures were pooled again so that the tetramers 1+2 and 3+4 were combined together and tetramers 5+6 and 7+8 were combined together. To each of the two resulting ligation mixtures was added an additional 2 μl of 10 mM ATP and 2 μl of T4 DNA ligase. The mixtures were incubated for 10 minutes at 37° and then at room temperature for 1 hour. Finally the entire ligation was pooled together, that is, both tubes were added together and an additional 8 μl of ligase were added to the ligation mixture and it was incubated for 10 minutes at 37° and then overnight at 4°. Following overnight ligation, one 10 μl aliquot was analyzed on a 8% polyacrylamide gel made up with 7M urea. A band could be discerned adjacent to the 242 base pair HpaII marker and indicated that the ligation was complete. An 8% polyacrylamide gel was made which also contained 7M urea. The ligation mix was ethanol precipitated, dried and then taken up in 80 μl of 80% formamide. Half of this ligation mix was then loaded onto the preparative gel adjacent to a lane containing HpaII cut PBR322 markers. The gel was run until the xylene cyanol dye marker had reached the bottom of the gel. The gel was then removed from the electrophoresis apparatus and placed in a film cassette next to a piece of Kodak X-Ray film. The bands were visualized by developing the film and the band just above the HpaII 242 marker on the adjacent lane was removed with the 244 base pair band expected for the completely ligated section I of fibroblast growth factor. This gel slice was extruded through a syringe into an eppendorf tube and covered with Maxam Gilbert Gel Elution solution and incubated overnight at 37°. The contents of the tube were then filtered through a glass fiber filter placed in a syringe barrel and the supernatant was extracted three times with N-butanol and ethanol precipitated. The dried pellet was then taken up in 200 µl of TE and reprecipitated with ethanol after removing the polyacrylamide residue which was centrifuged in the bottom of the tube. The ethanol precipitated sample contained about 37,000 counts per minute which corresponded to about 1.5pm of duplex based upon the radioactivity corresponding to the oligomers required for this ligation. These 1.5pm were then dissolved in 20 µl of ligation buffer containing $3 \times 10^7$ counts per minute of radiolabelled ATP. A 1/4 µl aliquot was removed and spotted on a DE81 strip. Then 1 µl of kinase was added and the tube was incubated at 37° for 45 minutes. At this point, 1/4 µl was removed from the tube and spotted on a second DE81 strip. Both strips were then eluted with 0.35 M ammonium formate and then cut into sections and counted on a liquid scintillation counter. The before and after strips clearly showed that counts were incorporated at the origin, therefore, the kination reaction was driven to completion by the addition of with 1 µl of 10mm ATP and incubation for 30 minutes longer at 37°. Then the kination was boiled for 5 minutes and slowed cooled to room temperature.

Fibroblast Growth Factor Section II was assembled in a similar manner. 200pm of each of the 12 oligonucleotides represented in Table IV were measured into eppendorf tubes and speed-vacuum to dryness. The

## TABLE IV

### FGF OLIGOMERS, SECTION II

| | |
|---|---|
| AATTCGGTACCTGGCTATGAAAGAAGACGGTCGTCTGCTGG | 17 |
| CTTCTAAGTGTGTTACTGACGAATGTTTCTTTTTCGAACG | 18 |
| TCTGGAATCTAACAACTACAACACTTACAGATCTCGTAAA | 19 |
| TACTCTTCCTGGTATGTAGCTCTGAAACGTACTGGTCAGT | 20 |
| ACAAACTGGGTCCGAAGACTGGTCCGGGTCAGAAAGCTATCC | 21 |
| TGTTTCTGCCGATGTCTGCTAAATCTTAATAGCTCGAGA | 22 |
| GAAGCCAGCAGACGACCGTCTTCTTTCATAGCCAGGTACCG | 23 |
| CAGACGTTCGAAAAAGAAACATTCGTCAGTAACACACTTA | 24 |
| AGTATTTACGAGATCTGTAAGTGTTGTAGTTGTTAGATTC | 25 |
| TTGTACTGACCAGTACGTTTCAGAGCTACATACCAGGAAG | 26 |
| AAACAGGATAGCTTTCTGACCCGGACCAGTCTTCGGACCCAGT | 27 |
| AGCTTCTCGAGCTATTAAGATTTAGCAGACATCGGCAG | 28 |

drying was repeated with 100 µl of 80% ethanol. A kinase mix was prepared which contained 24 µl of 10 × ligation buffer, 2 µl of radiolabelled ATP ($1.5 \times 10^7$ counts/minute/µl), 0.5 µl of 10mM ATP, 20 µl of kinase and 193 µl of water giving the total volume of 240 µl in the kinase mix. 20 µl of this mixture were added to each of the tubes, containing oligonucleotides 18-27, respectively, to be kinased. Tubes containing oligonucleotides 17 and 28 were given ligation buffer only. The tubes were then incubated for 45 minutes at 37° at which time a 1/4 µl aliquot was removed from each tube and spotted onto DE81 strips. The DE81 strips were then eluted with 0.35M ammonium formate and checked with a liquid scintillation counter to determine the number of counts at the origin. The counter showed that the kination was proceeding and at that point 1 µl of 10mM of ATP was added to each tube and the tubes were incubated for an additional 45 minutes at 37°. The tubes were boiled for 5 minutes and then centrifuged and combined to form duplexes. Oligonucleotide #23 was combined with #17 (duplex #17), #24 with #18 (duplex #18), #25 with #19 (duplex #19), #26 with #20 (duplex #20), #27 with #21 (duplex #21), and #28 with #22 (duplex #22). These duplex mixtures were then boiled and slow cooled to room temperature over a period of 1 hour. The duplexes were then combined to form tetramers. Duplexes 17+18 were combined to form tetramer 17, duplexes 19+20 were combined to form tetramer 19, and duplexes 21+22 were combined to form tetramer 21. These were annealed at 37° for 10 minutes. To each tetrameric mixture were added 2 µl of 10mM ATP and 2 µl of T4 DNA ligase. The 3 ligations were incubated overnight at 4°. At this point 4 µl aliquots were removed from each of the three tubes containing the tetramers and run on a 10% polyacrylamide gel made with 7M urea. Autoradiography of the gel showed that the ligation was proceeding. Tetramers 17 and 19 were pooled and 4 µl of ligase were added along with 4 µl of 10mM ATP. The 8 piece ligation was then incubated at 37° for 15 minutes and then at 4° at 6 hours before adding the last tetramer to the ligation mixture. At this point tetramer 21 was added to the octameric ligation mixture and the entire ligation was incubated at 37° for 15 minutes. 5 µl of ligase were added and 5 µl of 10mM ATP were added at 37° for 15 minutes. 5 µl of ligase were added and 5 µl of 10mM ATP were added to the ligation mixture, and the entire ligation was incubated overnight at 4°. A check of the full ligation on an 8% polyacrylamide gel made with 7M urea showed a prominent band at 242 base pairs as expected. The ligation mix was phenol extracted and ethanol precipitated before loading onto a prep gel. 1/2 of the ligation mix was loaded onto an 8% 7M urea gel and the 242 base pair product was visualized by autoradiography and removed.

## EXAMPLE 2

### Cloning and Expression of Bovine basic Fibroblast Growth Factor

The bovine bFGF gene was synthesized in two sections as described in Example 1. Each section was cloned into M13mp18 for sequence verification before assembly into an expression vector, pCFM1156. In preparation for the cloning of Section I, M13mp18 was digested with a threefold excess of EcoRI and XbaI for 2 hrs. The reaction was terminated by extraction with an equal volume of phenol followed by extraction with chloroform and precipitation with 2.5 volumes of ethanol. The DNA pellet was washed with ethanol, dried under vacuum and dissolved in 10 mM tris, 0.1 mM EDTA, pH 7.4. Ligation was carried out by incubation of 0.06 pmole of the M13mp18 vector prepared as described with 0.3 pmole of the synthetic FGF Section I in 50 mM tris (pH=7.4), 10 mM MgC12, 10mM DTT, 1 mM spermidine, 1 mM ATP, 100 ug/ml BSA, and 1 unit T4 ligase for 4 hrs. at 14 degrees C. JM109 host cells were made competent by centrifugation of cells from an exponentially growing culture, suspension in ice -cold 50 mM CaCl$_2$ at a concentration of 1.2 OD/ml for 20 minutes, followed by recentrifugation and resuspension of the cells in the same solution at a concentration of 12 OD/ml. Aliquots of the ligation mixture (0.1-10 µl) were added to 200 µl aliquots of the competent host cells and allowed to stand on ice for 40 minutes. The contents of each tube was then added to 200 µl of fresh JM109, 3 ml of molten L-top agar containing 10 µl of 100 mM IPTG and 50 µl of 2% X-Gal. This mixture was plated on an L-plate and incubated overnight at 37°C. Four of the resulting clear plaques were picked from the plates and grown in 10 ml cultures using JM109 as the host strain. Single strand phage DNA was prepared from these cultures and sequenced by the dideoxy method using M13 universal primer. One of these four had the desired sequence and was saved for assembly of the FGF gene into the expression vector.

Section II of the manufactured FGF gene was cloned into M13mp18 for sequencing using the same method as for section I. In this case, the M13mp18 vector was digested with EcoRI and HindIII (3-fold excess) in order to accommodate the sticky ends of section II. For the ligation, 0.025 pmole of M13mp18 vector was mixed with 0.075 pmole of the phosphorylated synthetic FGF section II and incubated 4 hours, 14°C as before. Transformation using the same CaCl$_2$ method resulted in clear plaques as for Section I, but since a high background of clear plaques was present on the control plates, further selection by hybridization was carried out. Several plaques were grown using JM109 host as described before and supernatants containing phage DNA were dotted on nitrocellulose filters. Oligonucleotides 18 and 24 used in the synthesis of Section II were radiolabeled with phosphorous-32 ATP using polynucleotide kinase and were used to probe these filters. Two positive-screening clones were selected and sequenced as before. One of these clones had the expected sequence and was used in the assembly of the gene into pCFM1156.

## EXAMPLE 3

### Assembly of bovine bFGF Gene in the Expression Vector pCFM1156

Double-stranded replicative form DNA for the section I and II M13 clones was prepared. 500 ml cultures of each phage in JM109 host were grown and the cells harvested by centrifugation. Cells were then resuspended in 15% sucrose, 0.05 M tris, 0.05 M EDTA, and 1 mg/ml lysozyme and incubated on ice for 25 minutes. RNAse was added to 0.1 mg/ml and incubation on ice continued for 10 more minutes. An equal volume of 0.1% triton X-100, 50 mM tris, 50 mM EDTA was added and incubation on ice continued for another 10 minutes. These lysates were then centrifuged for 60 minutes at 39000xg and the clear supernatant saved. Ethidium bromide was added to 1 mg/ml and cesium chloride was added to give a density of 1.55 g/ml. This solution was centrifuged for 18 hours at 45,000 rpm in a Vti-50 rotor in order to reach equilibrium. The supercoil DNA band from each tube was visualized by UV light and collected with a syringe. The ethidium bromide was quickly removed by extraction with butanol and the CsCl was removed by extensive dialysis against 10 mM tris, 0.1 mM EDTA. DNA stocks prepared in this way were used for further cloning.

Although numerous vectors may be employed to express this DNA, an expression vector having a regulated promoter and temperature inducible copy number control gene is preferred in order to maximize product yields. The expression plasmid pCFM1156 used in this example may readily be constructed from a plasmid pCFM836, the construction of which is described in published European Patent Application No. 136,490. pCFM836 is first cut with NdeI and then blunt-ended with T4 polymerase such that both existing NdeI sites are destroyed. Next, the vector is digested with ClaI and SacII to remove an existing polylinker before ligation to a substitute polylinker as illustrated in Table V. This substitute polylinker may be constructed according to the procedure of Alton, et. al., supra. Control of expression in the expression pCFM1156 plasmid is by means of a foreshortened lambda $P_L$ promoter, which itself may be under the control of a $C_{1857}$ repressor gene (such as is provided in E. coli strain K12 ΔHtrp).

The expression vector pCFM1156 was digested (2-fold excess) with XbaI and HindIII in preparation for ligation with FGF sections I and II. Section I and II DNA stocks, prepared as described above, were digested (2-fold excess) with either XbaI and KpnI (section I) or KpnI and HindIII (section II). All three digests were loaded onto a 1.2% sea-plaque agarose gel made in 50 mM tris-acetate and electrophoresed for 3 hours at 60 volts. The gel was stained with 1 ug/ml ethidium bromide solution and the bands were visualized under UV light. The linearized vector band and the section I and II bands were excised from the gel with a scalpel, placed in separate tubes and melted at 70°C for 15 minutes. Five microliters of the molten gel containing linearized vector was added to 10 µl each of the slices containing sections I and II and the mixture equilibrated to 37°C. The molten gel was mixed quickly with an equal volume of ice-cold 2X ligase buffer containing 2 mM ATP, and 0.5 unit T4 ligase and incubated overnight at 14 degrees C. Aliquots of this ligation mix were transformed into frozen competent FM6 host strain using a transformation protocol described by Hanahan, J. Mol. Biol. 166, 557-580, (1983), grown 2.5 hours to allow expression of kanamycin resistance, and plated on L-plates containing 20 ug/ml kanamycin. Plates were incubated at 28 degrees C overnight. Colonies were replica plated onto nitrocellulose filters and the master plate was saved. Colonies on the filters were grown to about 1mm diameter at 28 degrees and then placed at 37 degrees overnight to increase plasmid copy number. The filters were screened by hybridization with radiolabeled oligonucleotide 18 (from gene synthesis) at 65 degrees C in 6X SSC buffer. Twenty-five positive clones were obtained four were selected and grown in 500 ml cultures. Replicative form DNA was prepared as described for sections I and II using a cleared lysate procedure followed by CsCl equilibrium density gradient centrifugation. These four clones were sequenced

## TABLE V

```
 1   ATCGATTTGATTCTAGAAGGAGGAATAACATATGGTTAACGCGTTGGAATTCGGTACCAT
     TAGCTAAACTAAGATCTTCCTCCTTATTGTATACCAATTGCGCAACCTTAAGCCATGGTA

     1 ClaI, 12 XbaI, 29 NdeI, 35 HincII, Hpal, 39 MluI, 47  EcoRIl,
       53 HgiCl KpnI, 57 NcoI StyI,

61   GGAAGCTTACTCGAGGATCCGCGGATAAATAAGTAACGATCC
     CCTTCGAATGAGCTCCTAGGCGCCTATTTATTCATTGCTAGG

     63 HindIll, 70 Aval XhoI, 75 BamHI Xho2, 79 Sac2,
```

directly using the expression vector's double stranded form as a template for the dideoxy sequencing reactions and all four were found to have the correct sequence. One of these was chosen to be utilized in the expression of bovine bFGF and is hereinafter referred to as pCFM1156/bFGF. The DNA sequence for the bovine bFGF gene thus constructed in the pCFM1156/bFGF vector is represented in Table I.

EXAMPLE 4

### Expression and Purification of Bovine bFGF

### Expression

An overnight culture of the production strain was grown at 28°C in L-broth containing 20 µg/ml kanamycin and was used to inoculate an 8-liter fermentation batch. The 8-liter batch media contained 40g yeast extract, 40g glucose, 10g sodium chloride, and appropriate buffer salts, vitamin solution, and trace metals. A dual feed protocol was used. The initial feed (1 liter) contained 450g glucose plus appropriate vitamins and salts. After growth to 20 OD (optical density units at 600 nm) a second feed was begun at a rate of 200 ml/hr and the temperature was shifted to 42°C. This feed solution contained 200g/liter bactotryptone, 100g/liter yeast extract, and 100g/liter glucose. Growth was continued for 6 hours at 42°C with the cell concentration reacing 50 OD at harvest.

### Purification

E. coli cells were broken in water by Gaulin homogenizer, and centrifuged at 4.2K for 40 min with a J6B centrifuge. When analyzed by SDS-PAGE, FGF appeared in both pellet and supernatant, the protein in the supernatant being 60-70%. The pellet was, therefore, discarded and the supernatant was purified using ion exchange chromatography. The supernatant, after titration to pH 7.4 with Tris-base, was made 1 mM DTT and mixed with a CM-Sepharose resin equilibrated with 40 mM Tris-HCl/1 mM DTT/pH 7.4. The resin was then washed batchwise with the same buffer, and eluted column-wise with a linear NaCl gradient from 0 to 0.7M. A single peak around 0.5M was pooled based on SDS-PAGE analysis. The pool was titrated to pH 8.2 with Tris-base, diluted three-fold with cold water, and loaded onto a CM-Sepharose in 40 mM Tris-HCl/1 mM DTT/pH 8.2. The column was washed with 0.15M NaCl and eluted with a linear NaCl gradient of 0.15 to 0.5M. A major peak between two small peaks was pooled and found to be approximately 95% pure with a small amount of dimer when analyzed by non-reducing SDS-PAGE. The yield was approximately 90%.

The pool was immediately titrated to approximately pH 5 with 1 M NaOAc/pH 4 to prevent the bovine bFGF product from oxidation and then directly loaded on a Sephadex G-75 column in 20 mM Na citrate/0.1M NaCl/pH 5, resulting in a single peak eluting between a shoulder (corresponding to dimer) and a peak for small compounds (such as DTT). The fractions in the peak were pooled and stored at 4°C, -20°C or lyophilized. The yield in this gel filtration was nearly 100%. Approximately 400 mg of bovine bFGF was obtained from 560g of cell paste.

### EXAMPLE 5

### Preparation of Human bFGF

The procedure employed in this example is a modification of the procedure of Gilliam et. al., Gene, 12, p. 129-137 (1980). Human bFGF differs from bovine FGF by only two amino acids, at positions 113 (thr instead of ser) and 129 (ser instead of pro). Conversion of the bovine bFGF gene prepared in Example 1 to a gene prepared in Example 1 to a gene one coding for human bFGF requires only two nucleotide changes. These changes were made in the M13mp18/bFGF section II clone by site-directed mutagenesis using the following primers:

96-15) 5′ GACCAGTCTTCGAACCCAGTTTGTA 3′

96-16) 5′ CATACCAGGAAGTGTATTTACGAGA 3′

Ten pmole each of the M13 universal primer and the above primers were phosphorylated by incubation with 1 mM ATP and 10 units of polynucleotide kinase in 10 µl of 70 mM tris, 10 mM MgCl2, 5 mM DTT for 30 minutes at 37°C. Five pmole of each kinased oligonucleotide was mixed with about 0.5 ug of single stranded M13mp18/bFGF, heated to 65°, and allowed to renature by cooling to room temperature . To this template/primer mix was then added 1 µl of a solution 25 mM each in dATP, dCTP, dGTP, and TTP, 1 µl of 100 mM ATP, 2 units T4 ligase, and 8 units DNA PolI large fragment (Klenow). This mixture was incubated at 14°C for 4 hours. Aliquots of the ligation mix were transformed into JM101 as previously described and plated in 0.7% L-agar. Lifts onto nitrocellulose filters were performed on the resulting clear plaques and filters were screened by hybridization to radiolabeled oligonucleotides 96-15 and 96-16. Several positives were obtained using each probe, but none with both probes. One of the positives from 96-15 was chosen, single strand DNA was prepared and the procedure followed as before except only oligonucleotide 96-16 was used as a mutagenic primer. This experiment yielded several plaques which hybridized with both 96-15 and 96-16. Four of these were picked and single-strand DNA grown for sequencing; all four clones contained the correct sequence. The DNA sequence of the human bFGF gene, thus prepared is represented in Table II. E. coli derived human recombinant bFGF may be expressed and purified in accordance with the processes described in Example 4. The amino acid sequence for the human recombinant bFGF is represented in Table VI.

### EXAMPLE 6

Characterization of Bovine bFGF

Activity: The activity of bovine bFGF was examined in [3H] thymidine incorporation on 3T3 cells. All the preparations, stored at 4°C, and -20°C and lyophilized, showed a dose-dependent activity with a protein concentration from 20-210 pg/ml for half maximal activity depending on the particular strain of 3T3 cells utilized in the assay.

General Characteristics of the Final Product

260/280 ratio      ~2.0
LAL      <0.6EU/ml (0.623 FGF mg)
DNA      <20 pg/ml (0.623 FGF mg)
Extinction coefficient      1.3 for 0.1% protein
Purity      >95%

Stability

When a bovine bFGF preparation was incubated at 4°C at different pH, bovine bFGF showed formation of polymers composed of more than one bFGF molecule at pH ≧5.9 due to inter-chain disulfide bonds and degradation at pH ≦ 4.0 due to acid instability of Asp-Pro bond. Based on this information, the pH 5 buffer was selected. This stability data suggest that free -SH

## TABLE VI

### Human basic Fibroblast Growth Factor

```
1                              10                                  20
MetProAlaLeuProGluAspGlyGlySerGlyAlaPheProProGlyHisPheLysAsp

                               30                                  40
ProLysArgLeuTyrCysLysAsnGlyGlyPhePheLeuArgIleHisProAspGlyArg

                               50                                  60
ValAspGlyValArgGluLysSerAspProHisIleLysLeuGlnLeuGlnAlaGluGlu

                               70                                  80
ArgGlyValValSerIleLysGlyValCysAlaAsnArgTyrLeuAlaMetLysGluAsp

                               90                                 100
GlyArgLeuLeuAlaSerLysCysValThrAspGluCysPhePhePheGluArgLeuGlu

                               110                                120
SerAsnAsnTyrAsnThrTyrArgSerArgLysTyrThrSerTrpTyrValAlaLeuLys

                               130                                140
ArgThrGlyGlnTyrLysLeuGlySerLysThrGlyProGlyGlnLysAlaIleLeuPhe

                146
LeuProMetSerAlaLysSer
```

groups present in the final product tend to form inter-chain, rather than intra-chain, disulfide bond. The bovine bFGF preparation is apparently monomeric when determined by a Sephadex G-75 gel filtration.

EXAMPLE 7

HUV-EC Bioassay for FGF

HUV endothelial cells used in this experiment were isolated by Judith A. Berliner by the method of Gimbrone et al. (Gimbrone, MA, Cotran, R.S., Folkman, J. Human Vascular Endothelial Cells in Culture. The Journal of Cell Biology. 1974 Vol. 60 pgs. 673-684). Cells were maintained by subculturing into culture flasks coated with 0.1% gelatin in phosphate buffered saline, and 10 ug/ml fibronectin (Boehringer Mannheim) in media minus fetal bovine serum for 30 minutes each, consecutively. Cells were released with 0.0125% trypsin-0.005% EDTA and passed 1:2 or 1:3 once a week. The maintenance media used was MCDB105 (Irvine Scientific) with penicillin G (10 units/ml) streptomycin (10 ug/ml), fetal bovine serum (20%, hyclone), L-glutamine (2mM), sodium pyruvate (1mM), heparin (40 µg/ml, 170 units/mg) and endothelial cell growth supplement (ECGS, 40 ug/ml Collaborative Research). Cells were grown in a 2% $CO_2$ incubator.

The following experiment was carried out to compare the growth sustaining and mitogenic activities of three different forms of FGF on HUV endothelial cells. 1) E. coli derived recombinant bovine basic FGF (rbFGF); 2) bovine acidic FGF (aFGF, particularly purified from bovine brain); 3) bovine basic FGF (n-bFGF purified from bovine pituitary glands, Sigma).

One hundred cells were plated per well into four 24 well plates, using only the center 8 wells. Cells in each 24 well plate were grown in the presence of only one of the above growth factors or no growth factor in place of n-bFGF in the above maintenance media.

The cells were fed three and six days after the initial seeding with their same growth factor. Ten days after the seeding, cultures were analyzed by crystal violet straining.

The results in Table VII illustrate that there are 20% and 32% more colonies in the 8 wells which contained rbFGF then colonies in the wells containing either aFGF or n-bFGF, respectively. Also of interest is the fact that 75% of the total colonies arising from culture with rbFGF were 0.5 mm and larger, while only 55% and 51% of the colonies grown with aFGF and n-bFGF, respectively were that size.

These results indicate that rbFGF seems to be a better growth and proliferative factor than either aFGF or n-bFGF for HUV endothelial cells. A control containing no growth factor was also evaluated.

## Table VII

| Growth Factor | # of Colonies /8 Wells | # Colonies $\geq$0.5mm/8 Wells |
|---|---|---|
| rbFGF | 351 | 265 |
| aFGF | 293 | 160 |
| n-bFGF | 265 | 134 |
| Control | 8 | 0 |

EXAMPLE 8

FGF Bioassay on NIH 3T3 Cells

Cells used for this assay are NIH3T3 cells from ATCC. The cells are grown in DME medium with penicillin G (10 µg/ml), streptomycin (10 µg/ml), and calf serum (10%). The cells are passed 1:40, two times a week. On day 1 of assay, subconfluent cultures are trypsin dispersed and plated into 24 well plates at a concentration of $2 \times 10^4$ cells per ml, 1 ml per well in the above growth media.

On day 5, the media is replaced with DME with penicillin streptomycin and 5% human platelet poor plasma (cleared heparinized serum), 1 ml per well. Cells should be confluent at this point. On day 6, experimental and control samples of FGF are added to the media in volumes no greater than 100 µl.

Eighteen hours later, the cells are pulsed for 1 hour with 1 ml of DME with 5% calf serum and 2 µCi of 3H-Me thymidine at 37°C. The cells are then washed one time each with 1 ml of PBS and 5% trichloroacetic acid, both at 4°C. Plates are allowed to air dry for 30 minutes after which 1 ml of 0.25M NaOH is added to each well. After one hour at room temperature, the contents of each well are transferred to a separate counting vial containing 10 ml of aquasol II. Samples are counted for 1 minute through the 0-397 window of the LS 7,500 scintillation counter.

FGF standards are made from a stock with the concentration of 600 µg/ml in a sodium citrate buffer of pH5. The range of standards used is 5 pg to 1,000 pg per ml. The standard of lowest concentration which gives maximal 3H-thymidine uptake is 500 pg. An average of 140-210 pg gives the half maximal incorporation of labelled thymidine.

Controls: diluent 50 µl
  no additions

Duplicates of 50 µl each

diluent = DMEM + 0.1% BSA (Miles)
  + 0.01% NP40 + pen/strept + L-glutamine (2mM)
1/2 max is 140 - 210 pg

## Example 9

### Construction of the human bFGF gene

Conversion of the bovine bFGF gene prepared in Example 1 to a gene encoding for human bFGF was accomplished by oligo site directed mutagenesis.

The segment to be modified was first cloned into the phage vector M13mp18 and transformed into E.coli JM101 for growth and preparation of single stranded phage DNA (Messing, J. et al., Vol. 9, Nucl. Acid Res. pp.309-321 (1981)). Approximately 0.5 ug of template DNA was mixed with 5 pmol universal M13 sequencing primer and 5 pmol of each mutagenic primer, heated to 65 degress for 3 minutes and allowed to slow cool. The annealed template-primer was then mixed with ATP, a dNTP mix, DNA PolI large fragment, and ligase followed by incubation at 15 degrees for 4 hours. Aliquots of this reaction mix were transformed into competent JM101 cells and plated in 0.7% L-agar. Plaques containing mutant phage were selected by hybridization of replica nitrocellulose filters with 32P-labeled mutagenic primer. Single strand DNA was prepared from positive-screening plaques and its sequence verified using the dideoxy chain-termination method. The amino acid changes made and the corresponding mutagenic primers used were:

    ser-113 to thr-113) 5′ GACCAGTCTTCGAACCCAGTTTGTA 3′
     pro-129 to ser-129) 5′ CATACCAGGAAGTGTATTTACGAGA 3′

The primers correspond to the antisense strand of the bFGF gene.

## Example 10

### Fermentation Production of FGF

The fermentation production of FGF is as follows:

A vial of culture is removed from a secured culture storage -70°C freezer. The vial is defrosted at room temperature and inoculated under a laminar flow hood into Fernbach flasks, each containing Luria broth (Luria broth: bactotryptone 10 g/L, yeast extract 5 g/L, NaCl 5 g/L).

The inoculated flasks are then shaken for 10-16 hours at approximately 28°C. A fermenter containing batch media (Table VIII) which has been autoclaved according to written standard operating procedure, is inoculated by aseptic transfer of the contents of the Fernbach flasks. The agitation rate, temperature, pH and dissolved oxygen are set as specified in the manufacturing formula. pH is automatically maintained with phosphoric acid and ammonium hydroxide. Dissolved oxygen is maintained at the specific level by increases in the agitation rate, air flow rate, and/or back pressure.

Samples are removed aseptically, for cell density measurement, at set intervals. Feed medium #1 (Table VIII) is fed in over time at specified flow rates according to the cell density. At a specified cell density, the temperature is raised to appoximately 42°C to induce product synthesis. Feed medium #1 is immediately replaced with feed medium #2 (Table I). Feed medium #2 is maintained at one specific feeding rate to the end of fermentation. Approximately six hours after the increase in temperature, the fermenter is cooled and the cells in the fermenter are harvested by centrifugation.

## Table VIII

| | Batch medium | Feed medium #1 | Feed medium #2 |
|---|---|---|---|
| **Chemicals** | | | |
| Bactotryptone | 200 g/L | 200 g/L | |
| Yeast extract | 5 g/L | 100 g/L | 100 g/L |
| $(NH_4)_2SO_4$ | 3.75 g/L | | |
| $K_2HPO_4$ | 7 g/L | | |
| $KH_2PO_4$ | 8 g/L | | |
| NaCl | 1.25 g/L | | |
| Glucose | 5 g/L | 25 g/L | 50 g/L |
| $MgSO_4 \cdot 7H_2O$ (1 M) | 4 ml/L | | |
| Trace metal soln. | 2 ml/L | | |
| Vitamin solution | 2 ml/L | | |
| $FeCl_3 \cdot$ citrate soln. | 6 ml/L | | |

Example 11

Purification of human bFGF

E.coli cells containing the synthetic bFGF genes in pCFM1156 were grown as described above in Example 4. After disruption of the cells and low speed centrifugation, the bFGF was found both in the supernatant and pellet fractions. Purification from the pellet requires solubilization by denaturants followed by refolding to obtain active material. These steps can be avoided by purification from the supernatant fraction as described below. This fraction was applied to a CM-sepharose column in 40 mM Tris-HCl, pH 7.4 and eluted with a linear NaCl gradient. The fractions containing bFGF were then bound to the same resin, but in 40 mM Tris-HCl, pH 8.2 and again eluted with a linear NaCl gradient. In these two chromatographies, 1mM DTT was included to prevent oxidation, which otherwise resulted in the formation of intermolecular disulfide bonds. The protein was further purified by gel filtration using a Sephadex G-75 column in 20 mM sodium citrate, 0.1M NaCl, pH 5.0. Initial attempts to purify FGF from E. coli cells showed that dimer is readily formed when 1 mM DTT is not included throughout the purification process. Purification of the human bFGF was carried out in essentially the same manner as for the bovine material as set forth in Example 4. No difference between human and bovine bFGF was noted in any of the purification steps. When examined on SDS-PAGE under reducing conditions, the bFGFs gave a major band at 16,500 daltons corresponding to the monomeric molecular weight and minor bands at higher molecular weights which probably represent dimer and tetramer forms. When run under non-reducing conditions, more contamination by the higher molecular weight bands is apparent. Amino acid sequence analysis of the purified bFGF revealed that methionine had been cleaved from most of the material, yielding 70% proline, 13% alanine, and only 17% methionine on the N-terminus. As does the natural material, recombinant bFGF exhibits a strong affinity for heparin, eluting from heparin sepharose columns at approximately 1.5-2.0 M NaCl (data not shown).

Example 12

Characterizations of human bFGF

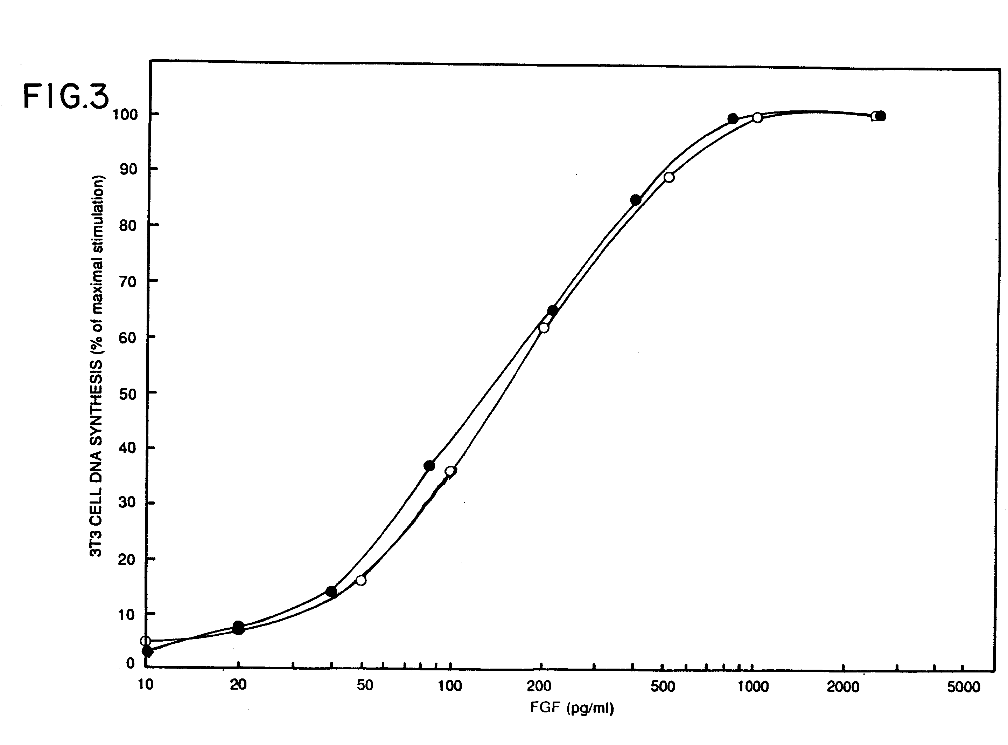

The activity of human bFGF was examined in [3H] thymidine incorporation on 3T3 cells as described in Example 8. NIH 3T3 cells were obtained from ATCC. The cells were grown in DME supplemented with 10% calf serum, 10 u/ml penicillin and 10 ug/ml streptomycin. Cells were passed 1:40 two times per week. On day 1 of the assay, subconfluent cultures were trypsin dispersed and plated into 24-well plates at a concentration of 20,000 cells/ml, 1 ml per well in the above media. On day 5, the media is replaced with DME containing penicillin, streptomycin, and 5% human platelet poor plasma, 1 ml/well. On day 6, experimental samples were added to the media in volumes no greater than 100 μl. Eighteen hours later, cells were pulsed for 1 hour with 1 ml DME containing 5% calf serum and 2 uCi of tritiated thymidine at 37 degrees. The cells were then washed once each with 1 ml PBS and 5% trichloroacetic acid, both at 4 degrees. Plates were allowed to air dry for 30 minutes, then 1 ml of 0.25 M NaOH was added and allowed to remain for 1 hour at room temperature. The contents of each well was then transferred to a separate vial containing 10 ml of Aquasol II and counted in a liquid scintillation counter. As shown in Fig. 3, recombinant human bFGF show essentially identical potency in this assay, as recombinant bovine bFGF, producing half-maximal stimulation of DNA synthesis at a dose of about 150-200 pg/ml.

Example 13

HUV-EC BioAssay for human FGF

The HUVE cell assay is described in Example 7. Human umbilical vein endothelial cells used in this experiment were a gift from Dr. Judith A. Berliner. Cells were grown in flasks prepared by coating with 0.1% gelatin in phosphate buffered saline for 30 minutes followed by 10 ug/ml fibronectin in media without fetal bovine serum (FBS) for 30 minutes. The maintenance media used was MCDB105 (Irvine Scientific) supplemented with 10 u/ml penicillin G, 10 ug/ml streptomycin, 20% FBS, 2mM L-glutamine, 1mM sodium pyruvate, 40 ug/ml heparin (170 u/mg), and 40 ug/ml endothelial cell growth supplement (ECGS, Collaborative Research). Cells were plated into 24-well plates and grown in the maintenance media containing ECGS, or in the same media with recombinant human bFGF or no growth factor replacing the ECGS. The cells were fed 3 and 6 days after the initial seeding, at which time fresh growth factor was added. Ten days after seeding, the cells were stained with crystal violet and counted. Addition of recombinant human FGF resulted in extensive cell proliferation in comparison with controls containing no growth factor. No significant differences between bovine and human bFGF was observed.

Numerous modifications are variations in the practice of the invention are expected to occur to those skilled in the art upon consideration of the foregoing illustrative examples. Consequently, the invention should be considered as limited only to the extent reflected by the appended claims.

The features disclosed in the foregoing description, in the following claims and/or in the accompanying drawings may, both separately and in any combination thereof, be material for realising the invention in diverse forms thereof.

## Claims

1. A DNA sequence for use in securing expression in an E. coli host cell of a polypeptide product having at least a part of the primary structural conformation and one or more of the biological properties of naturally-occurring human basic fibroblast growth factor, said DNA sequence selected from among:

(a) the DNA sequence set out in Table II or the complimentary strands;

(b) DNA sequences which hybridize to the DNA sequences defined in (a) or fragments thereof; and

(c) DNA sequences which, but for the degeneracy of the genetic code, would hybridize to the DNA sequences defined in (a) and (b).

2. An E. coli host cell transformed with a DNA sequence according to claim 1 in a manner allowing the host cell to express said polypeptide product.

3. A biologically functional plasmid or viral DNA vector including a DNA sequence according to claim 1.

4. An E. coli host cell stably transformed with a DNA vector according to claim 3.

5. A process for producing a polypeptide having part or all of the primary structural conformation and one or more of the biological properties of naturally occurring human basic fibroblast growth factor, said process comprising:

a) growing under suitable nutrient conditions, E. coli host cells transformed with a DNA plasmid vector wherein the DNA plasmid vector comprises a DNA sequence coding for E. coli host expression of a polypeptide having part or all of the primary structural conformation and one or more of the biological properties of basic fibroblast growth factor, a regulated promoter sequence, and a temperature inducible copy number control gene;

b) isolating desired polypeptide products of the expression of DNA sequence in said vector;

c) purifying the desired polypeptide product.

6. A process according to Claim 1 wherein the desired polypeptide product is purified using a non-heparin containing chromatography system.

7. A process according to Claim 1 wherein said DNA sequence is a DNA sequence according to Claim 1.

8. A DNA plasmid vector comprising a DNA sequence coding for E. coli host expression of a polypeptide having part or all of the primary structural conformation and one or more of the biological properties of human basic fibroblast growth factor, a regulated promoter sequence, and a temperature

17

inducible copy number control gene.

9. A method for purifying recombinant basic fibroblast growth factor expressed from an E. coli host strain containing a plasmid encoding for basic fibroblast growth factor comprising chromatographing the recombinant basic fibroblast growth factor following isolation from the E. coli host strain using a non-heparin containing chromatographic column.

# bFGF-GENE ASSEMBLY AND CLONING

FIG.I

# FIG.2

```
                              Met Pro Ala Leu Pro Glu Asp Gly Gly
    TCTAGAAGGAGGAATAACAT ATG CCA GCT CTG CCA GAA GAT GGT GGA
      Xba I

    10                                              20
    Ser Gly Ala Phe Pro Pro Gly His Phe Lys Asp Pro Lys Arg Leu
    TCC GGT GCT TTC CCG CCA GGT CAT TTC AAA GAT CCG AAA CGT CTG

                              30
    Tyr Cys Lys Asn Gly Gly Phe Phe Leu Arg Ile His Pro Asp Gly
    TAC TGC AAA AAC GGT GGT TTT TTC CTG CGT ATC CAT CCG GAT GGT

    40                                              50
    Arg Val Asp Gly Val Arg Glu Lys Ser Asp Pro His Ile Lys Leu
    CGT GTT GAT GGT GTA CGT GAG AAA TCT GAT CCG CAT ATC AAA CTG

                              60
    Gln Leu Gln ala Glu Glu Arg Gly Val Val Ser Ile Lys Gly Val
    CAG CTG CAA GCT GAA GAG CGT GGT GTA GTT TCT ATT AAA GGT GTA

    70                                              80
    Cys Ala Asn Arg Tyr Leu Ala Met Lys Glu Asp Gly Arg Leu Leu
    TGT GCT-AAC CGG TAC CTG GCT ATG AAA GAA GAC GGT CGT CTG CTG
                   Kpn I


                              90
    Ala Ser Lys Cys Val Thr Asp Glu Cys Phe Phe Phe Glu Arg Leu
    GCT TCT AAG TGT GTT ACT GAC GAA TGT TTC TTT TTC GAA CGT CTG


    100                                             110
    Glu Ser Asn Asn Tyr Asn Thr Tyr Arg Ser Arg Lys Tyr |Ser| Ser
    GAA TCT AAC AAC TAC AAC ACT TAC AGA TCT CGT AAA TAC |TCT| TCC
                                                        |A  |
                                                        |Thr|

                              120
    Trp Tyr Val Ala Leu Lys Arg Thr Gly Gln Tyr Lys Leu Gly |Pro|
    TGG TAT GTA GCT CTG AAA CGT ACT GGT CAG TAC AAA CTG GGT |CCG|
                                                            |T  |
                                                            |Ser|

    130                                             140
    Lys Thr Gly Pro Gly Gln Lys Ala Ile Leu Phe Leu Pro Met Ser
    AAG ACT GGT CCG GGT CAG AAA GCT ATC CTG TTT CTG CCG ATG TCT

            147
    Ala Lys Ser End End
    GCT AAA TCT TAA TAG CTCGAGAAGCTT
                            Hind III
```

FIG.3

3T3 CELL DNA SYNTHESIS (% of maximal stimulation)

FGF (pg/ml)